# EUROPEAN PATENT SPECIFICATION

(11) **EP 0 319 481 B1**
(45) Date of publication and mention of the grant of the patent: **03.02.1993**
(21) Application number: 88810822.2
(22) Date of filing: 30.11.1988
(51) Int. Cl.: C07D 249/08, C07D 233/56, C07D 401/06, C07D 405/06, C07D 409/06, C07C 255/35, A01N 43/653, A01N 43/50

(54) **Azole compounds**
Azolverbindungen
Composés azoliques

(30) Priority: 02.12.1987 GB 8728235
(43) Date of publication of application: 07.06.1989
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Schneider, Rupert, CH-4125 Riehen (CH)

(56) References cited:
- EP-A- 0 061 840
- EP-A- 0 145 294
- EP-A- 0 234 683

## Description

The present invention relates to novel 2-cyano-2-aryl-1-(azole-1-yl)-ethane derivatives, their use, compositions for facilitating their use and the preparation of novel compounds of the invention.

The invention provides compounds of formula I 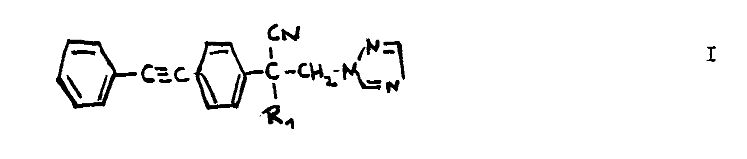
wherein R₁ is C₁₋₃ alkyl or allyl
The compounds of the invention contain one or more chiral centres. Such compounds are generally obtained in the form of racemic or diastereomeric mixtures. However, these and other mixtures can if desired be separated either completely or partly into the individual isomers or desired isomer mixtures by methods known in the art.

Where R is C₁₋₃ alkyl, it is in particular CH₃ or C₂H₅.

EP-A-0 234 683 discloses compounds which differ from those of the present invention in that the phenyl group is substituted in the 2-position by an OR group.

EP-A-0 145 294 discloses compounds which differ from those of the present invention in that the phenyl group has a 4-halo substituent.

EP-A-0 061 840 discloses compounds which differ from those of the present invention in that R₁ in the formula (I) of the present application, is optionally substituted phenyl instead of C₁₋₃alkyl or allyl, and in that none of both phenyl groups is stated to (optionally) bear an alkyne group.

The compounds of the present invention have a surprisingly interesting botryticidal activity.

The compounds of formula I are obtained by
a) reacting a compound of formula II 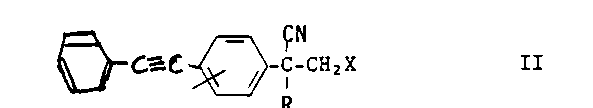 wherein R and R₁ are as defined above,
   and X is halogen,
   with a compound of formula III 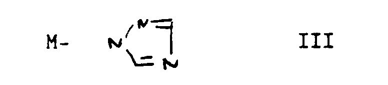 wherein M is a metal, or
b) by reacting a compound of formula IV 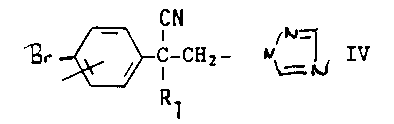 wherein R₁ is as defined above,
   with an acetylene of formula V

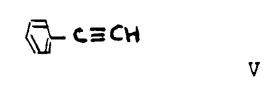

The reaction of a compound of formula II with a compound of formula III is conveniently effected in a suitable solvent, e.g. dimethylsulfoxide. The reaction is preferably carried out with heating, e.g. at a temperature in the range of from 100° to 150°C.

M is preferably an alkalimetal, e.g. Na.

X is preferably chlorine or bromine.

The reaction of a compound of formula IV with a compound of formula V is conveniently effected in the presence of catalytic amounts of a palladium catalyst such as bis[triphenylphosphine]palladium dichloride or tetrakis-(triphenylposphin)-palladium(O) and copper(I)iodide in an amine such as diethylamine, triethylamine or piperidine.

A suitable reaction temperature is between ambient temperature and reflux temperature of the reaction mixture, preferably at enhanced temperature, e.g. between 80° and 100°C.

The compounds of the invention are isolated from the reaction mixture in which they are formed by established procedures. They may be obtained in free form or in salt or metal complex form, e.g. as acid addition salt with an organic or inorganic acid such as hydrochloride, or as alcoholate e.g as Na ethanolate, or in metal complex form, e.g. with a metal such as copper and zinc, and with anions such as chloride, sulphate and nitrate.

Compounds of formula II may e.g. be obtained by substitution of 4-bromobenzylcyanide by R₁, CH₂Br and the group phenyl-ethinyl in appropriate order, in a manner known per se, e.g. by preparing 2-cyano-2-(4-bromo-phenyl)-ethylbromide from 4-bromo-benzylcyanide, substituting then the 4-bromo substituent therein by the phenyl-ethinyl group R-, e.g. analogous to process b) described hereinbefore, and reacting the thus obtained acetylene compound of formula VI 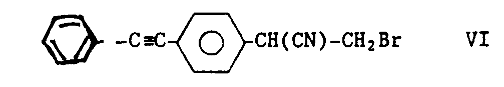
with a compound of formula VII

R₁Br VII

wherein R₁ is as defined above, in the presence of a phase-transfer catalyst.

Insofar as the production of any starting material is not particularly described, these compounds are known, or may be produced and purified in accordance with known processes, or in a manner analogous to processes described herein or to known processes.

The compounds of formula I in free form or in agriculturally acceptable salt or complex form are useful as fungicides in the combatting of phytopathogenic fungi. Their advantageous fungicidal activity is established by in vivo tests with test concentrations from about 0.0008 to 0.05% against Uromyces appendiculatus (bean rust) on pole beans, against other rust fungi (such as Hemileia, Puccinia) on coffee, wheat, against Erysiphe cichoracearum on cucumber and against other powdery mildew fungi (E. graminis f.sp. tritici, E. graminis f.sp. hordei, Podosphaera leucotricha, Uncinula necator) on wheat, barley, apple, grape vine. Further interesting activities are i.a. observed in vitro against Ustilago maydis and in vivo against Rhizoctonia solani on cotton and in particular and in particular against Botrytis strains on beans, tomato, peperoni and grape vine. Many of the compounds of the invention have an excellent plant tolerance and a systemic action. The compounds of the invention are therefore indicated for treatment of plant, seeds and soil to combat phytopathogenic fungi, e.g. Basidiomycetes of the order Uredinales (rusts) such as Puccinia spp, Hemileia spp, Uromyces spp; Ascomycetes of the order Erysiphales (powdery mildew) such as Erysiphe spp, Podosphaera spp, and Uncinula spp; as well as Phoma; Helminthosporium; Deuteromycetes such as Pyricularia, Pellicularia (=Corticium), Thielaviopsis, Stereum spp and Botrytis.

The amount of compound of the invention to be applied, will depend on various factors such as the compound employed, the subject of the treatment (plant, soil, seed), the type of treatment (e.g. drenching, sprinkling, spraying, dusting, dressing), the purpose of the treatment (prophalactic or therapeutic), the type of fungi to be treated and the application time.

In general, satisfactory results are obtained, if the compounds of the invention are applied in an amount of from about 0.0005 to 2.0, preferably about 0.01 to 1 kg/ha, in the case of a plant or soil treatment; e.g. 0.04 to 0.500 kg of active ingredient (a.i.) per ha in crops such as cereals, or concentrations of 4 to 50 g of a.i. per hl in crops such as fruits, vineyards and vegetables (at an application volume of from 300 to 1000 l/ha - depending on the size or leaf volume of the crop - which is equivalent to an application rate of approxi-mately 40-500 g/ha). The treatment can, if desired, be repeated, e.g. at intervals of 8 to 30 days.

The preferred compounds of formula I have an activity against Botrytis which is similar to that observed with iprodione, said compounds of formula I being also active against iprodione resistent strains.

Where the compounds of the invention are used for seed treatment, satisfactory results are in general obtained, if the compounds are used in an amount of from about 0.05 to 0.5, preferably about 0.1 to 0.3 g/kg seeds.

The term soil as used herein is intended to embrace any conventional growing medium, whether natural or artificial.

The compounds of the invention may be used in a great number of crops, such as soybean, coffee, ornamentals (i.a. pelargonium, roses), vegetables (e.g. peas, cucumber, celery, tomato and bean plants), sugarbeet, sugarcane, cotton, flax, maize (corn), vineyards, pomes and stone fruits (e.g. apple, pears, prunes) and in cereals (e.g. wheat, oats, barley, rice).

In view of their good crop tolerance many of the compounds of the invention are particularly indicated for fungicidal treatments where a favourable crop tolerance is desirable or essential, e.g. in fruit crops such as apples and grapes. Various compounds of the invention possess also a favourable curative activity.

The invention also provides fungicidal compositions, comprising as a fungicide a compound of the invention in free form, or in agriculturally acceptable salt or complex form in association with a agriculturally acceptable diluent (hereinafter diluent). They are obtained in conventional manner, e.g. by mixing a compound of the invention with a diluent and optionally additional ingredients, such as surfactants.

The term diluents as used herein means liquid or solid agriculturally acceptable material, which may be added to the active agent to bring it in an easier or better applicable form, resp. to dilute the active agent to a usable or desirable strength of activity. Examples of such diluents are talc, kaolin, diatomaceous earth, xylene or water.

Especially formulations used in spray form,such as water dispersable concentrates or wettable powders, may contain surfactants such as wetting and dispersing agents, e.g. the condensation product of formaldehyde with naphthalene sulphonate, an alkylarylsulphonate, a lignin sulphonate, a fatty alkyl sulphate, an ethoxylated alkylphenol and an ethoxylated fatty alcohol.

In general, the formulations include from 0.01 to 90% by weight of active agent, from 0 to 20% agriculturally acceptable surfactant and from 10 to 99.99% diluent(s). Concentrated forms of composition, e.g. emulsion concentrates, contain in general from about 2 to 90%, preferably from between 5 and 70% by weight of active agent. Application forms of formulation contain in general from 0.0005 to 10% by weight of a compound of the invention as active agent typical spray-suspensions may, for example, contain from 0.0005 to 0.05, e.g. 0.0001, 0.002 or 0.005% by weight of active agent.

In addition to the usual diluents and surfactants, the compositions of the invention may comprise further additives with special purposes, e.g. stabilizers, desactivators (for solid formulations or carriers with an active surface), agents for improving the adhesion to plants, corrosion inhibitors, anti-foaming agents and colorants. Moreover, further fungicides with similar or complementary fungicidal activity, e.g. sulphur, chlorothalonil, euparen, a guanidine fungicide such as guazatine, dithiocarbamates such as mancozeb, maneb, zineb, propineb, trichloromethane sulphenylphthalimides and analoges such as captan, captafol and folpet, benzimidazoles such as carbendazim, benomyl, or other beneficially-acting materials, such as insecticides may be present in the formulations.

Examples of plant fungicide formulations are as follows:

### a. Wettable powder Formulation

10 Parts of a compound of the invention are mixed and milled with 4 parts of synthetic fine silica, 3 parts of sodium lauryl sulphate, 7 parts of sodium lignin sulphonate and 66 parts of finely divided kaolin and 10 parts of diatomaceous earth until the mean particle size is about 5 micron. The resulting wettable powder is diluted with water before use to a spray liquor which may be applied by foliar spray as well as by root drench application.

### b. Granules

Onto 94.5 parts by weight of quartz sand in a tumbler mixer are sprayed 0.5 parts by weight of a binder (non-ionic tenside) and the whole thoroughly mixed. 5 parts by weight of a compound of the invention are then added and thorough mixing continued to obtain a granulate formulation with a particle size in the range of from 0.3 to 0.7 mm. The granules may be applied by incorporation into the soil adjacent to the plants to be treated.

### c. Emulsion Concentrate

10 Parts by weight of a compound of the invention are mixed with 10 parts of weight of an emulsifier and 80 parts by weight of toluene. The concentrate is diluted with water to the desired concentration.

### d. Seed Dressing

45 Parts of a compound of the invention are mixed with 1.5 parts of diamyl phenoldecaglycolether ethylene oxide adduct, 2 parts of spindle oil, 51 parts of fine talcum and 0.5 parts of colorant rhodanin B. The mixture is ground in a contraplex mill at 10,000 rpm until an average particle size of less than 20 microns is obtained. The resulting dry powder has good adherance and may be applied to seeds, e.g. by mixing for 2 to 5 minutes in a slowly turning vessel.

The following examples further illustrate the present invention. All temperatures are in centigrade. Rf values are obtained by thin layer chromatography on silica gel, unless otherwise specified.

### FINAL PRODUCTS

### Example 1

### 2-Cyano-2-[4-(phenylethinyl)phenyl]-1-(1H-1,2,4-triazol-1-yl)-butane

A mixture of 3.1 g (0.01 mol) of 2-cyano-2-(4-bromophenyl)-1-(1H-1,2,4-triazol-1-yl)-butane, 1.6 g (0.015 mol) of phenylacetylene, 0.05 g of tetrakis-(triphenyl-phosphin)palladium-(O), 0.1 g of copper (I)iodide and 0.2 g triphenylphosphine in 50 ml of triethylamine are stirred for 2 hours at 90° under a blanket of nitrogen. The reaction mixture is concentrated by evaporation at the water jet and the residue stirred with 150 ml of toluene. The thus obtained solution together with a solid phase still contained therein is washed with water (3x), dried and evaporated to give an oily residue which slowly solidifies. It may be purified by recrystallisation from a mixture of petrol ether/toluene (7:3) or by column chromatography with silicagel employing methylenchlorid/methanol (98:2) as eluant.

The thus obtained title product (compound 1) has a m.p. of 127-129°.

### Example 2

Following the procedure of Example 1 each of the compounds of Formula I listed in Table A is obtained from the corresponding 4-bromophenyl compound of formula IV and phenyl-ethinyl compound.

**TABLE A**

| (Compounds of formula I wherein R-C≡C is in the 4-position) | | |
|---|---|---|
| Cmpd. | R₁ | Rf/m.p. |
| 1 | C₂H₅ | 127-129° |
| 2.1 | CH₃ | 104° |
| 2.2 | n-C₃H₇ | 63° |
| 2.3 | i-C₃H₇ | 138° |
| 2.4 | -CH₂-CH=CH₂ | 85° |

### Example 3

### 2-Cyano-2-[4-(phenylethinyl)phenyl]-1-(1H-1,2,4-triazol-1-yl)-butane

2.1 g (0.03 mol) triazole are dissolved in 16 ml dimethylsulfoxide, 1.2 g (0.03 mol) pulverised sodium hydroxide are added with stirring and the mixture heated to 110°. At this temperature a solution of 3.8 g (0.011 mol) 2-cyano-2-[4-(phenylethinyl)-phenyl]-butylbromide in 16 ml of dimethylsulfoxide is added dropwise and the mixture stirred for two more hours at 110°. After cooling the reaction mixture is poured into water, extracted with diethyl ether, washed with water, dried with anhydrous Na₂SO₄ and evaporated under water jet vacuum. The residue is chromatographed on a silica gel column employing CH₂Cl₂/CH₃OH 98:2 as eluant. The thus obtained oily title compound solidifies and is then recrystallised from ligroine to give colourless crystals, m.p. 129°.

### INTERMEDIATES

### Example 4

### 2-Cyano-2-(4-bromophenyl)-1-(1H-1,2,4-triazol-1-yl)-butane

a) A mixture of 35 g (0.156 mol) of 2-(4-bromophenyl)-propylcyanide, 50 ml methylenebromide, 50 ml of 50% sodiumhydroxyde and 4 g of benzyl-triethylammoniumbromide is heated for 4 hours under reflux (95°). The reaction mixture is cooled, diluted with 200 ml of CH₂Cl₂. The organic phase is separated off and washed with water (3 times with 100 ml each). The solvent is evaporated and the residue recrystallised from hexane/diethylether 95:5 to give 2-cyano-1-(4-bromophenyl)-butylbromide (m.p. 87°).
b) 2.2 g (0.032 Mol) of 1,2,4-triazole and 1.3 g (0.032 mol) pulverised NaOH in 30 ml of dimethylsulfoxide are heated at 110°. To this mixture are added, within a few minutes, at 110° a solution of 2-cyano-2-(4-bromophenyl)butylbromide in 10 ml of dimethylsulfoxide. The reaction mixture is stirred at 110° for another 90 minutes, poured into 600 ml of water and extracted several times with diethylether. The united ether phases are washed with water and dried with magnesiumsulfate. Thereafter the ether is evaporated at the water jet and residues of dimethylsulfoxide at high vacuum to give the title compound (solid; m.p. 68-70°, Rf=0.45 employing CH₂Cl₂/CH₃OH 95:5 as eluant).

### Example 5 [4-(Phenylethinyl)phenyl]-acetonitrile

A mixture of 49 g (0.25 mol) 4-bromobenzylcyanide, 30 g (0.3 mol) phenylacetylene, 0.1 g di(triphenylphosphine)-palladium(II)-dichloride, 0.1 g copper(I)iodide and 0.2 g triphenylphosphine in 300 ml triethylamine are heated for 20 hours under a blanket of N₂ and vigorously stirred. The mixture is then evaporated under water jet vacuum, the residue heated in hexane and the undissolved solid sucked off and dried. The thus obtained title compound is employed as such in the following reaction step.

### Example 6 2-[Phenylethinyl)phenyl]-butyronitrile

To 1.8 g (0.075 mol) NaH in 30 ml dimethylformamide are added dropwise, at 0° to 10°, a solution of 14 g (0.064 mol) [4-(phenylethinyl)phenyl]acetonitrile in 30 ml dimethylformamide and the mixture stirred until the H₂ gas development ceases.

Then are added dropwise, at 0° to 10°, 9.8 g (0.09 mol) of ethylbromide in 20 ml of dimethylformamide and the mixture is stirred for 8 more hours at room temperature. After addition of a few ml of water to destroy excessive NaH, the reaction mixture is poured into 1000 ml of water, extracted with diethyl ether, the ether extracts washed with water, dried and evaporated and the residue chromatographed over a silica gel column, employing hexane:acetone 95:5 as an eluant. The title compound has a Rf-value of 0.22 and solidifies after evaporation of the solvent (m.p. 75°).

### Example 7 2-Cyano-2-[4-(phenylethinyl)phenyl]butyl bromide

To a solution of 5 g (0.021 mol) 2-[4-(phenylethinyl)phenyl]-butyronitrile in 65 ml CH₂Br₂ are added, with stirring, 64 g 50% (0.8 mol) NaOH and 5.5 g (0.025 mol) benzyltriethylammoniumchloride. The mixture is stirred for 5 hours at 95°, cooled and 250 ml CH₂Cl₂ are added. The organic phase is separated off, washed, dried and evaporated in vacuum. The thus obtained title compound is kept for one hour at 70-80° under high vacuum and crystallises from toluene: ligroine 1:2, m.p. 107°.

## Claims (Claims for the following Contracting State(s): AT, BE, CH, DE, FR, GB, GR, IT, LI, NL)

1. Compounds of formula I 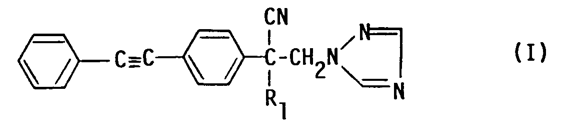 wherein R₁ is C₁₋₃alkyl or allyl,
in free form, salt or metal complex form.

2. The compound of Claim 1, wherein R₁ is CH₃, C₂H₅ or allyl.

3. The compound of claim 2, wherein R₁ is CH₃.

4. The compound of claim 2, wherein R₁ is C₂H₅.

5. The compound of claim 2, wherein R₁ is allyl.

6. Process of preparing a compound of formula I, stated in Claim 1, which comprises
a) reacting a compound of formula II 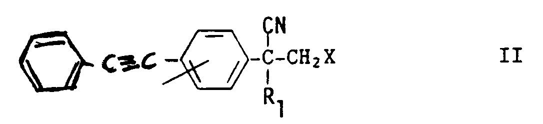 wherein R₁ is as defined in Claim 1,
with a compound of formula III 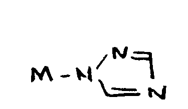 wherein M is a metal, or
b) by reacting a compound of formula IV 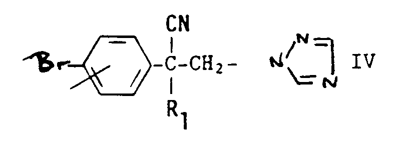 wherein R₁ is as defined in Claim 1,
with an acetylene of formula V 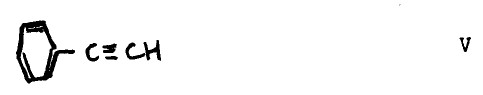 and isolating the thus obtained compounds of formula I in free form, salt form or metal complex form.

7. A fungicidal composition comprising a compound of formula I according to any one of Claims 1 to 6 in free form or in agriculturally acceptable salt or complex form.

8. A method of combatting phytopathogenic fungi comprising applying to such fungi or their locus a fungicidally effective amount of a compound claimed in any one of Claims 1 to 6 in free form or in agriculturally acceptable salt or complex form.

9. The compounds of formula II 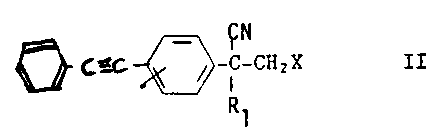 wherein
R₁ is as stated in Claim 1 and
X is halogen.

## Claims (Claims for the following Contracting State(s): ES)

1. Process for preparing compounds of formula I 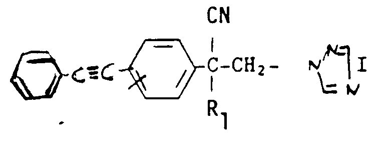 wherein R₁ is C₁₋₃alkyl or allyl
in free form, salt form or metal complex form, which comprises
a) reacting a compound of formula II 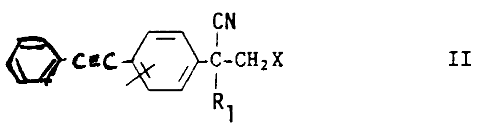 wherein R₁ is as defined in this Claim,
with a compound of formula III 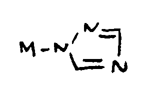 wherein M is a metal, or
b) by reacting a compound of formula IV 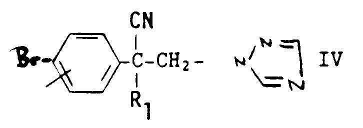 wherein R₁ is as defined in this Claim,
with an acetylene of formula V 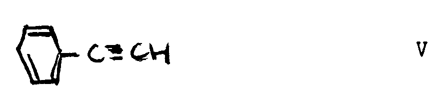 and isolating the thus obtained compounds of formula I in free form, salt form or metal complex form.

2. The process of Claim 1, wherein R₁ is CH₃, C₂H₅ or allyl.

3. The process of claim 2, wherein R₁ is CH₃

4. The process of claim 2, wherein R₁ is C₂H₅

5. The process of claim 2, wherein R₁ is allyl

6. A fungicidal composition comprising a compound of formula I as defined in any one of Claims 1 to 5 in free form or in agriculturally acceptable salt form or metal complex form.

7. A method of combatting phytopathogenic fungi which comprises applying to such fungi or their locus a fungicidally effective amount of a compound of formula I, defined in any one of Claims 1 to 5, in free form or in agriculturally acceptable salt form or metal complex form.

## Patentansprüche (Patentansprüche für folgende(n) Vertragsstaat(en): AT, BE, CH, DE, FR, GB, GR, IT, LI, NL)

1. Verbindungen der Formel I 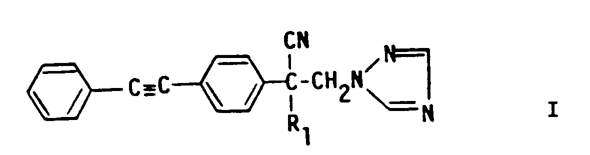 worin R₁ für C₁-C₃-Alkyl oder Allyl steht, in freier Form oder in Form eines Salzes oder Metallkomplexes.

2. Verbindung nach Anspruch 1, worin R₁ für CH₃, C₂H₅ oder Allyl steht.

3. Verbindung nach Anspruch 2, worin R₁ für CH₃ steht.

4. Verbindung nach Anspruch 2, worin R₁ für C₂H₅ steht.

5. Verbindung nach Anspruch 2, worin R₁ für Allyl steht.

6. Verfahren zur Herstellung einer Verbindung der Formel I gemäß Definition von Anspruch 1, dadurch gekennzeichnet, daß
a) eine Verbindung der Formel II 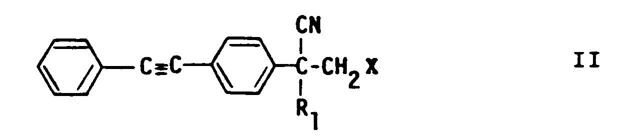 worin R₁ wie im Anspruch 1 definiert ist und X für Halogen steht, mit einer Verbindung der Formel III 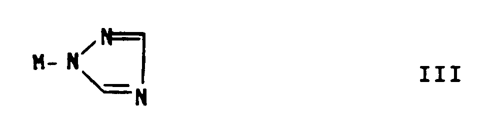 worin M ein Metall ist, umgesetzt wird oder
b) eine Verbindung der Formel IV 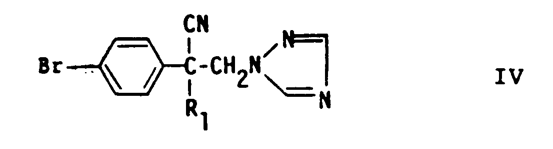 worin R₁ wie im Anspruch 1 definiert, mit einem Acetylen der Formel V 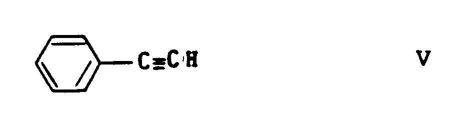 umgesetzt wird und
die so erhaltenen Verbindungen der Formel I in freier Form oder in Form eines Salzes oder Metallkomplexes isoliert werden.

7. Fungizide Zusammensetzung, dadurch gekennzeichnet, daß sie eine Verbindung der Formel I nach einem der Ansprüche 1 bis 6 in freier Form oder in Form eines landwirtschaftlich annehmbaren Salzes oder Komplexes enthält.

8. Verfahren zur Bekämpfung phytopathogener Pilze, dadurch gekennzeichnet, daß solche Pilze oder ihr Lebensraum mit einer fungizid wirksamen Menge einer Verbindung nach einem der Ansprüche 1 bis 6 in freier Form oder in Form eines landwirtschaftlich annehmbaren Salzes oder Komplexes behandelt werden.

9. Verbindungen der Formel II 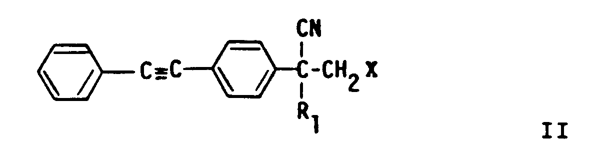 worin R₁ wie im Anspruch 1 angegeben definiert ist und X für Halogen steht.

## Patentansprüche (Patentansprüche für folgende(n) Vertragsstaat(en): ES)

1. Verfahren zur Herstellung von Verbindungen der Formel I 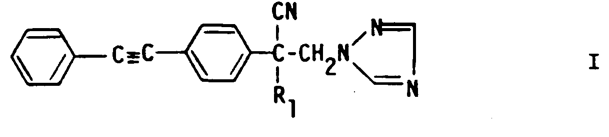 worin R₁ für C₁-C₃-Alkyl steht, in freier Form oder in Form eines Salzes oder Metallkomplexes, dadurch gekennzeichnet, daß
a) eine Verbindung der Formel II 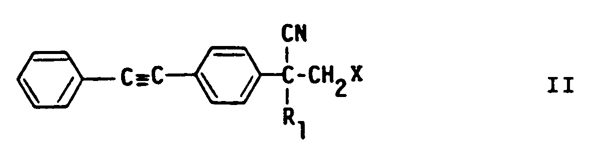 worin R₁ wie in diesem Anspruch definiert ist und X für Halogen steht, mit einer Verbindung der Formel III 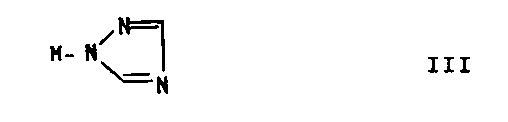 worin M ein Metall ist, umgesetzt wird oder
b) eine Verbindung der Formel IV 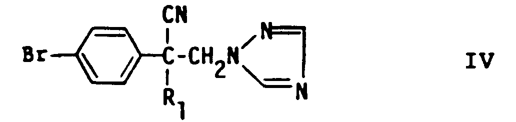 worin R₁ wie in diesem Anspruch definiert ist, mit einem Acetylen der Formel V 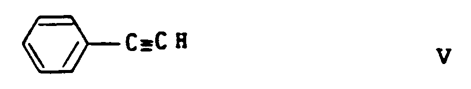 umgesetzt wird und
die so erhaltenen Verbindungen der Formel I in freier Form oder in Form eines Salzes oder Metallkomplexes isoliert werden.

2. Verfahren nach Anspruch 1, worin R₁ für CH₃, C₂H₅ oder Allyl steht.

3. Verfahren nach Anspruch 2, worin R₁ für CH₃ steht.

4. Verfahren nach Anspruch 2, worin R₁ für C₂H₅ steht.

5. Verfahren nach Anspruch 2, worin R₁ für Allyl steht.

6. Fungizide Zusammensetzung, dadurch gekennzeichnet, daß sie eine Verbindung der Formel I nach einem der Ansprüche 1 bis 5 in freier Form oder in Form eines landwirtschaftlich annehmbaren Salzes oder Komplexes enthält.

7. Verfahren zur Bekämpfung phytopathogener Pilze, dadurch gekennzeichnet, daß solche Pilze oder ihr Lebensraum mit einer fungizid wirksamen Menge einer Verbindung nach einem der Ansprüche 1 bis 5 in freier Form oder in Form eines landwirtschaftlich annehmbaren Salzes oder Komplexes behandelt werden.

## Revendications (Revendications pour l'(les) Etat(s) contractant(s) suivant(s): AT, BE, CH, DE, FR, GB, GR, IT, LI, NL)

1. Les composés de formule I 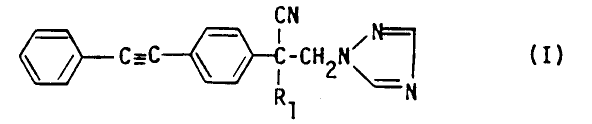 dans laquelle R₁ signifie un groupe alkyle en C₁-C₃ ou allyle,
sous forme libre, sous forme d'un sel ou sous forme d'un complexe métallique.

2. Le composé de la revendication 1, dans lequel R₁ signifie CH₃, C₂H₅ ou allyle.

3. Le composé de la revendication 2, dans lequel R₁ signifie CH₃.

4. Le composé de la revendication 2, dans lequel R₁ signifie C₂H₅.

5. Le composé de la revendication 2, dans lequel R₁ signifie un groupe allyle.

6. Un procédé de préparation d'un composé de formule I, spécifié à la revendication 1, qui comprend
a) la réaction d'un composé de formule II 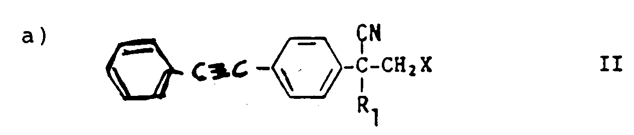 dans laquelle R₁ est tel que défini à la revendication 1,
avec un composé de formule III 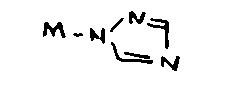 dans laquelle M signifie un métal, ou
b) la réaction d'un composé de formule IV 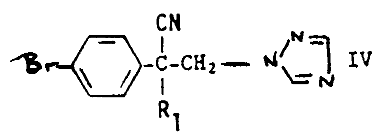 dans laquelle R₁ est tel que défini à la revendication 1,
avec un acétylène de formule V 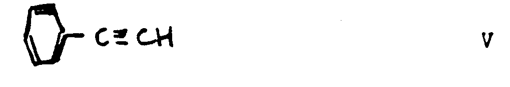 et l'isolement des composés de formule I ainsi obtenus, sous forme libre, sous forme de sels ou sous forme de complexes métalliques.

7. Une composition fongicide, comprenant un composé de formule I selon l'une quelconque des revendications 1 à 6, sous forme libre ou sous forme d'un sel ou d'un complexe acceptable en agriculture.

8. Une méthode pour combattre les champignons phytopathogènes, comprenant l'application à de tels champignons ou à leur habitat d'une quantité efficace du point de vue fongicide d'un composé spécifié à l'une quelconque des revendications 1 à 6, sous forme libre ou sous forme d'un sel ou d'un complexe acceptable en agriculture.

9. Les composés de formule II 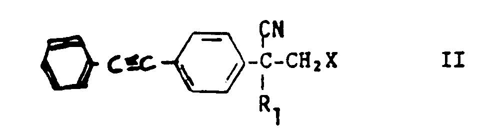 dans laquelle
R₁ est tel que spécifié à la revendication 1, et
X signifie un halogène.

## Revendications (Revendications pour l'(les) Etat(s) contractant(s) suivant(s): ES)

1. Un procédé de préparation des composés de formule I 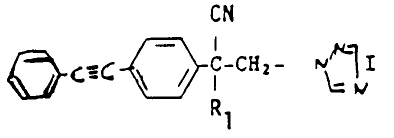 dans laquelle
R₁ signifie un groupe alkyle en C₁-C₃ ou allyle,
sous forme libre, sous forme d'un sel ou sous forme d'un complexe métallique, qui comprend
a) la réaction d'un composé de formule II 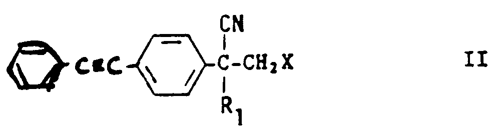 dans laquelle R₁ est tel que défini à la présente revendication,
avec un composé de formule III 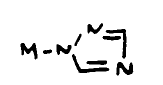 dans laquelle M signifie un métal, ou
b) la réaction d'un composé de formule IV 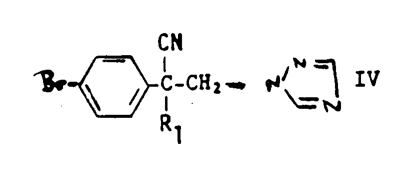 dans laquelle R₁ est tel que défini à la présente revendication,
avec un acétylène de formule V 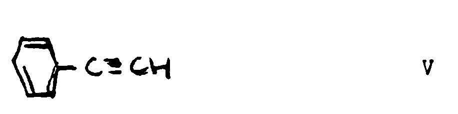 et l'isolement des composés de formule I ainsi obtenus, sous forme libre, sous forme de sels ou sous forme de complexes métalliques.

2. Le procédé de la revendication 1, dans lequel R₁ signifie CH₃, C₂H₅ ou allyle.

3. Le procédé de la revendication 2, dans lequel R₁ signifie CH₃.

4. Le procédé de la revendication 2, dans lequel R₁ signifie C₂H₅.

5. Le procédé de la revendication 2, dans lequel R₁ signifie un groupe allyle.

6. Une composition fongicide, comprenant un composé de formule I tel que défini à l'une quelconque des revendications 1 à 5, sous forme libre ou sous forme d'un sel ou d'un complexe métallique acceptable en agriculture.

7. Une méthode pour combattre les champignons phytopathogènes, qui comprend l'application à de tels champignons ou à leur habitat d'une quantité efficace du point de vue fongicide d'un composé de formule I, défini à l'une quelconque des revendications 1 à 5, sous forme libre ou sous forme d'un sel ou d'un complexe métallique acceptable en agriculture.
